(19) Europäisches Patentamt
European Patent Office
Office européen des brevets

(11) Publication number: 0 035 770
B1

(12) EUROPEAN PATENT SPECIFICATION

(45) Date of publication of patent specification: 30.12.86

(21) Application number: 81101645.0

(22) Date of filing: 06.03.81

(51) Int. Cl.[4]: A 61 K 31/71, A 61 K 31/73, A 61 K 45/06, A 61 K 47/00 // (A61K31/73, 31:71, 31:66, 31:60, 31:19)

# (54) An orally administered drug form comprising a glycosidic antibiotic and an adjuvant.

(30) Priority: 07.03.80 US 128100

(43) Date of publication of application:
16.09.81 Bulletin 81/37

(45) Publication of the grant of the patent:
30.12.86 Bulletin 86/52

(84) Designated Contracting States:
AT BE CH DE FR GB IT LI LU NL SE

(56) References cited:
EP-A-0 009 686
FR-A-2 379 285
GB-A-1 559 840
US-A-3 777 018

ROTE LISTE, 1961, Cantor, AULENDORF/WÜRTT. (DE), page 305, "Ell-Aknell Salbe"

The file contains technical information submitted after the application was filed and not included in this specification

(73) Proprietor: INTERx RESEARCH CORPORATION
2201 West 21st Street
Lawrence Kansas 66044 (US)

(72) Inventor: Higuchi, Takeru
2811 Schwarz Road
Lawrence Kansas 66044 (US)
Inventor: Nishihata, Toshiaki
2428 Redbud Lane
Lawrence Kansas 66044 (US)

(74) Representative: Abitz, Walter, Dr.-Ing. et al
Abitz, Morf, Gritschneder P.O. Box 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 035 770 B1

**Description**

Background of the Invention

*Field of the Invention*

The present invention relates to the oral delivery of macrolide or aminoglycoside antibiotics which by this route are poorly absorbed and more especially to the enhancement of this delivery by formulations which contain a hydroxyaromatic acid.

Description of the Prior Art

It is well known to the art that a number of antibiotics are only slowly absorbed from the gastrointestinal tract. Consequently, said antibiotic must be administered by the intravenous or intramuscular route or in excessively large oral doses in order to attain clinical efficacy. The β-lactam antibiotics is one class of antibiotics which is poorly absorbed by the oral route. Similarly, the glycosidic and related antibiotics, which are chemically characterized by a chemical structure which includes a carbohydrate moiety bonded to the remainder of the molecule by a glycosidic linkage, are poorly absorbed by the oral route. The glycosidic and related antibiotics, such as the macrolide, aminoglycoside, lincomycin, anthracycline and other chemically related antibiotics, are poorly absorbed from the gastrointestinal tract because of two properties, hydrophilicity and/or acid instability. The hydrophilic, polar nature of these antibiotics precludes their rapid absorption so that even the small percentage which is absorbed is subject to a long residency time in the acidic gastric environment. It is therefore clear that any factor which enhances the rate of absorption will demonstrate improved clinical efficacy.

Many attempts have been made to improve the oral absorption of glycosidic and related antibiotics. Acid instability is partially overcome by coating the antibiotic with an acid stable-base labile enteric coating. This procedure has achieved some success with the acid labile, macrolide antibiotic, erythromycin; however, this procedure still does not allow complete absorption. Other approaches center on the reduction of the hydrophilicity of the glycosidic and related antibiotics by preparing chemical derivatives which are more lipophilic, see Murphy, Antibiotic Annual 1953—1954, page 500; Stephens, Antibiotic Annual 1958—1959, page 346; and Jones, Journal of Medicinal Chemistry, Volume 15, page 631, 1972. These lipophilic esters of erythromycin show enhanced blood levels when administered to warm-blooded animals.

The many attempts to enhance the oral absorption of glycosidic and related antibiotics have met with limited success with the macrolide antibiotics such as erythromycin and oleandomycin and with the lincomycin antibiotics. In these cases specialty coatings and lipophilic chemical derivatives have demonstrated improved absorption from the gastrointestinal tract, but in most cases absorption is incomplete. When similar techniques were applied to the aminoglycoside and anthracycline antibiotics, little or no oral absorption was reported. There is no oral dosage form for either of these two important classes of antibiotics.

Thus, there exists a clear and present need for a novel method to enhance the oral absorption of glycosidic and related antibiotics. Said method would permit the oral use of the aminoglycoside and anthracycline antibiotics and provide an improved oral form for the macrolide and lincomycin antibiotics.

Summary of the Invention

Accordingly, a major object of this invention is to provide a class of agents which enhance the oral absorption of macrolide and aminoglycoside antibiotics.

Another object is to provide a process utilizing said class of agents to enhance the oral absorption of macrolide and aminoglycoside antibiotics.

Another object is to provide a stable drug form utilizing said class of agents which when administered orally will provide increased blood levels of the therapeutic agent.

Other objects, features and advantages of the invention will be apparent to those skilled in the art from the detailed description of the invention which follows.

All of the foregoing objects are readily attained by providing a method and drug form wherein the oral absorption of macrolide and aminoglycoside antibiotics is enhanced, the method comprising the steps of preparing a drug form suitable for oral delivery, and a drug form comprising an effective unit dosage amount of the macrolide or aminoglycoside antibiotic, a hydroxyaryl or hydroxyaralkyl acid of formula I or a salt thereof, the latter adjuvants being present in said drug form in an amount sufficient to be effective in enhancing the rate of the oral absorption of the antibiotic and a suitable pharmaceutically accepted excipient.

Detailed Description of the Invention

The present invention, generally relates to an orally administered drug form comprising a

therapeutically effective amount of a macrolide or aminoglycoside antibiotic and an adjuvant of the formula

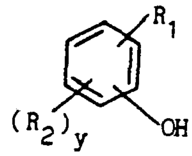

(I)

wherein $R_1$ is $CO_2H$, $(CH_2)COOH$,

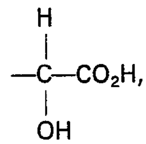

, $SO_3H$,

or a pharmaceutically acceptable salt thereof, wherein $R_2$ is OH, H, lower alkoxy radical having 1—10 carbon atoms, a lower alkyl radical having 1—10 carbon atoms, a halo radical, or a tri-halo lower alkyl radical having 1—5 carbon atoms, and wherein y is an integer of 1 or 2.

Specific examples of lower alkoxy radicals are methoxy, ethoxy, butoxy, and octyloxy. Specific examples of lower alkyl radicals are methyl, isopropyl, ethyl, t-butyl, n-butyl, and t-octyl. A specific example of a tri-halo lower alkyl radical is trifluoromethyl.

Specific adjuvants useful in our method and drug forms for enhancing oral absorption of polar bioactive agents include salicyclic acid, resorcylic acid, gentisic acid, and homovanillic acid. Such adjuvants are not considered novel *per se* and may be prepared by techniques known to those skilled in the art.

The amount of hydroxyaryl or hydroxyaralkyl acids or salt ester or amide thereof used in our method and drug forms may vary over a wide range; in general, the identity and the amount of the hydroxyaryl or hydroxyaralkyl acid or salt thereof is used in connection with drug in order to be effective in enhancing the absorption rate of the drug from the gastrointestinal compartment into the bloodstream. The effectiveness of the hydroxyaryl or hydroxyaralkyl acid adjuvants becomes significant at local concentration exceeding 0.01% at the absorption site. Their use at a dosage whereby their concentration at the absorption site exceeds 5% is not recommended because of the local irritating effect on the tissue.

The following combinations of macrolide or aminoglycoside antibiotics and hydroxyaryl or hydroxy aralkyl acids were given to beagle dogs by gavage. The urine was collected by catheterization and blood by venous puncture.

| Example | Antibiotic | Hydroxyaryl or Hydroxyaralkyl Acid |
|---|---|---|
| 1 | erythromycin | sodium salicylate |
| 2 | oleandomycin | salicylic acid |
| 3 | spiramicin | gentisic acid |
| 4 | kanamycin | resorcylic acid |
| 5 | neomycin | sodium gentisate |
| 6 | kanamycin | homovanillic acid |
| 7 | gentamicin | sodium salicylate |
| 8 | butirosin | sodium homovanillate |
| 9 | sisomycin | salicylic acid |
| 10 | netilmicin | sodium salicylate |

The drug forms of this invention are suitably administered in oral dosage form, such as by tablet or capsule, by combining the macrolide or aminoglycoside antibiotic in a therapeutic amount and the hydroxyaryl or hydroxyaralkyl acid or salt thereof in a sufficient quantity to be effective to enhance oral delivery with an oral pharmaceutically acceptable inert carrier, such as lactose, starch (pharmaceutical grade), dicalcium phosphate, calcium sulfate, Kaolin, mannitol and powdered sugar. In order to reduce the irritation in the stomach, the preferred dose form of the hydroxyaryl or hydroxyaralkyl acid should be a

pharmaceutically acceptable salt and the drug form should be designed to release the macrolide or aminoglycoside antibiotic and the hydroxyaryl or hydroxyaralkyl acid salt beyond the pylorus. In addition, when required, suitable binders, lubricants, disintegrating agents, and coloring agents can also be added. Typical binders include, without limitation, starch, gelatin, sugars such as sucrose, molasses, and lactose, natural and synthetic gums, such as acacia, sodium alginate, extract of Irish moss, carboxymethylcellulose, methylcellulose, and polyvinylpyrrolidone, polyethylene glycol, ethylcellulose and waxes. Typical lubricants for use in these dosage forms can include, without limitation, boric acid, sodium benzoate, sodium acetate, sodium chloride, leucine and polyethylene glycol. Suitable disintegrators can include, without limitation, starch, methylcellulose, agar, bentonite, cellulose and wood products, alginic acid, guar gum, citrus pulp, carboxymethylcellulose, and sodium lauryl sulfate. Optionally, if desired, a conventionally, pharmaceutically acceptable dye can be incorporated into the oral dosage unit form, e.g., any of the standard FD & C dyes.

Example I

Preparation of Sodium 2-hydroxy-5-methoxy benzenesulfonate

p-Methoxyphenol (12.4 g) was dissolved in chloroform (100 ml) and cooled in ice. Chlorosulfonic acid (11.6 g) was added dropwise to the stirred reaction mixture. The cooling bath was removed after the addition and stirring continued for 24 hours at room temperature. The chloroform was then evaporated off and the residue was vacuum dried to a hygroscopic light brown solid weighing 20.5 g which was 2-hydroxy-5-methoxy-benzenesulfonic acid. NMR ($CDCl_3$) 3.73 (3H, s, $OCH_3$), 6.8—7.2 (3H, m, aromatic *H*), and 9.86 (2H, broad s, O*H* and $SO_3H$). IR (film) 3500—2900, 1512, 1470, 1229, 1198, 996, 938 $cm^{-1}$.

The above sulfonic acid (10 g) was dissolved in water (10 ml) and poured into 75 ml of saturated sodium chloride solution. A white solid separated immediately. It was filtered and dried. Crystallization from water gave the pure sodium salt of 2-hydroxy-methoxybenzenesulfonic acid (6.6 g). NMR ($D_2O$), 3.83 (3H, s, $OCH_3$), 7.05 and 7.33 (3H, multiplets, aromatic). IR (KBr) 3260, 1518, 1440, 1300, 1280, 1240, 1210, 1905, 1045 $cm^{-1}$.

Example II

Typical preparation of enteric-coated tablets containing adjuvant

*300 mg Gentamycin Capsules*

Equal weights of gentamycin sulfate and sodium 5-methoxysalicylate were mixed and 600 mg of the mixture was used to fill No. 00 hard gelatin capsules. A small drop of water around the shell overlap was used to seal the capsules.

Coating:

The capsules were coated with 25 mg of pre-coat and 65 mg of enteric coating according to the coating procedure described below.

*Enteric Coating Procedure*

Tablets or capsules were placed in a coating pan containing baffles to provide adequate tumbling. A small amount of the coating solution was applied using an air sprayer and the solvents evaporated with a warm air supply directed into the coating pan. This procedure was repeated until the desired amount of coating material was applied. The amount of coating material was determined from the weight gain of a representative group of tablets.

Coating Solutions:

*Pre-coat:* A film of hydroxypropylmethylcellulose was applied to the tablets followed by an enteric coating.

*Enteric coat:* A film of hydroxypropylmethylcellulosephthalate was applied.

*Solutions:* A 5% by weight solution of hydroxypropylmethylcellulose and a 10% by weight solution of hydroxypropylmethylcellulosephthalate in ethanol:methylene chloride (1:1 by weight) were used as the coating solutions.

Example III

The following adjuvants or their corresponding acids can be used in addition to those previously mentioned in the example above and with the macrolide and aminoglycoside antibiotics previously mentioned.

Sodium 5-methoxysalicylate
Sodium salicylate
Sodium homovanilate
Sodium 2,5-dihydroxybenzoate
Sodium 2,4-dihydroxybenzoate
Sodium 3,4-dihydroxymandelate
Sodium 3-methoxy-4-hydroxymandelate
Sodium-5-methoxy-2-hydroxyphenylsulfonate
Sodium 3-methylsalicylate
Sodium 5-methylsalicylate
Sodium 5-tert-octylsalicylate

Sodium 3-tert-butyl-5-methylsalicylate
Sodium guaiacolsulfonate
Sodium 5-bromosalicylate
Sodium 3,5-dibromosalicylate
Sodium 5-iodosalicylate
Sodium 3,5-diiodosalicylate
Sodium 2-hydroxyphenylacetate
Sodium mandelate
Sodium phenyllactate
Sodium 2-hydroxyphenylmethanesulfonate
Sodium 5-trifluoromethyl-2-hydroxybenzoate
Sodium 3-methoxysalicylate
Sodium 5-octyloxysalicylate
Sodium 5-butoxysalicylate
Sodium 3,4-dihydroxyphenylacetate
Sodium 5-chlorosalicylate
Sodium 3,5-dihydroxybenzoate
Sodium 2-hydroxy-3-methoxybenzoate

Any skilled artisan concerned with the subject matter of this invention can prepare these oral dosage forms by simply referring to the oral dosage form preparatory procedure outlined in REMINGTON'S PHARMACEUTICAL SCIENCES, Fifteenth Edition (1975), pages 1576 through 1617 inclusive.

**Claims for the Contracting States: BE CH DE FR GB IT LI LU NL SE**

1. An orally administered drug form comprising a therapeutically effective amount of a macrolide or aminoglycoside antibiotic and an adjuvant of the formula

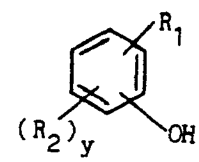

wherein $R_1$ is $CO_2H$, $(CH_2)COOH$,

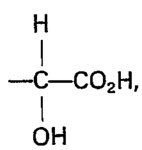
, $SO_3H$,

or a pharmaceutically acceptable salt thereof, wherein $R_2$ is OH, H, lower alkoxy radical having 1—10 carbon atoms, a lower alkyl radical having 1—10 carbon atoms, a halo radical, or a tri-halo lower alkyl radical having 1—5 carbon atoms, and wherein y is an integer of 1 or 2.

2. The drug form of claim 1 wherein said macrolide antibiotic is erythromycin, oleandomycin, spiramycin or kitasamycin.

3. The drug form of claim 1 wherein said aminoglycoside antibiotic is gentamicin, kanamycin, neomycin, paromycin, streptomycin, tobramycin, viomycin, butirosin, netilmicin or sisomycin.

4. The drug form of claim 1 whereein said adjuvant is sodium salicylate or sodium methoxysalicylate.

**Claims for the Contracting State: AT**

1. A method of enhancing the rate of absorption of an orally administered macrolide or aminoglycoside antibiotic into the blood stream, said method comprising the steps of preparing a drug form capable of being orally absorbed, said drug form comprising a therapeutically effective dosage amount of a macrolide or aminoglycoside antibiotic and an adjuvant of the formula

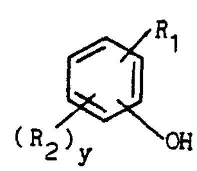

wherein $R_1$ is $CO_2H$, $(CH_2)COOH$,

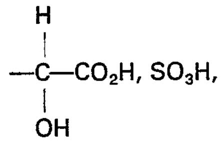

or a pharmaceutically acceptable salt thereof, wherein $R_2$ is OH, H, lower alkoxy radical having 1—10 carbon atoms, a lower alkyl radical having 1—10 carbon atoms, a halo radical, or a tri-halo lower alkyl radical having 1—5 carbon atoms, and wherein y is an integer of 1 or 2, said adjuvant being present in said drug form in a sufficient amount to be effective in enhancing said oral absorption rate.

2. The method of claim 1 wherein said macrolide antibiotic is erythromycin, oleandomycin, spiramicin or kitasamycin.

3. The method of claim 1 wherein said aminoglycoside antibiotic is gentamicin, kanamycin, neomycin, paromycin, streptomycin, tobramycin, viomycin, butirosin, netilmicin or sisomycin.

4. The method of claim 1 wherein said adjuvant is sodium salicylate or sodium methoxysalicylate.

**Patentansprüche für die Vertragsstaaten: BE CH DE FR GB IT LI LU NL SE**

1. Eine Arzneimittelformulierung zur oralen Verabreichung, enthaltend eine therapeutisch wirksame Menge eines Macrolid- oder Aminoglycosidantibiotikums und einen Hilfsstoff der Formel

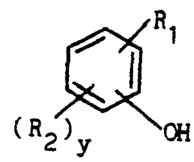

worin $R_1$ $CO_2H$, $(CH_2)COOH$,

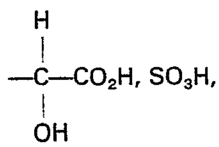

oder ein pharmazeutisch annehmbares Salz davon ist, worin $R_2$ OH, H, ein Niedrigalkoxyrest mit 1 bis 10 Kohlenstoffatomen, ein Niedrigalkylrest mit 1 bis 10 Kohlenstoffatomen, ein Halogenrest, oder ein Trihalogenniedrigalkylrest mit 1 bis 5 Kohlenstoffatomen ist, und worin y eine ganze Zahl von 1 oder 2 ist.

2. Die Arzneimittelformulierung des Anspruchs 1, wobei das genannte Macrolidantibiotikum Erythromycin, Oleandomycin, Spiramycin oder Kitasamycin ist.

3. Die Arzneimittelformulierung des Anspruchs 1, wobei das genannte Aminoglycosidantibiotikum Gentamicin, Kanamycin, Neomycin, Paromycin, Streptomycin, Tobramycin, Viomycin, Butirosin, Netilmicin oder Sisomycin ist.

4. Die Arzneimittelformulierung des Anspruchs 1, wobei der genannte Hilfsstoff Natriumsalicylat oder Natriummethoxysalicylat ist.

**Patentansprüche für den vertragstaat: AT**

1. Ein Verfahren zur Erhöhung der Rate der Absorption eines oral verabreichten Macrolid- oder Aminoglycosidantibiotikums in den Blutstrom, welches Verfahren die Stufen der Herstellung einer oral absorbierbaren Arzneimittelformulierung umfaßt, wobei die genannte Arzneimittelformulierung eine therapeutisch wirksame Dosierungsmenge eines Macrolid- oder Aminoglycosidantibiotikums und einen Hilfsstoff der Formel

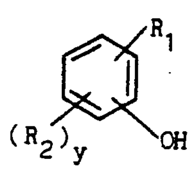

worin

$R_1$ $CO_2H$, $(CH_2)COOH$,

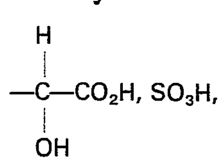

oder ein pharmazeutisch annehmbares Salz davon ist, worin $R_2$ OH, H, ein Niedrigalkylrest mit 1 bis 10 Kohlenstoffatomen, ein Niedrigalkylrest mit 1 bis 10 Kohlenstoffatomen, ein Halogenrest, oder ein Trihalogenniedrigalkylrest mit 1 bis 5 Kohlenstoffatomen ist, und worin y eine ganze Zahl von 1 oder 2 ist, enthält, und der genannte Hilfstoff in der genannten Arzneimittelformulierung in einer ausreichenden Menge vorliegt, um bei der Erhöhung der genannten oralen Absorptionsrate wirksam zu sein.

2. Das Verfahren des Anspruchs 1, wobei das genannte Macrolidantibiotikum Erythromycin, Oleandomycin, Spiramicin oder Kitasamycin ist.

3. Das Verfahren des Anspruchs 1, wobei das genannte Aminoglycosidantibiotikum Gentamicin, Kanamycin, Neomycin, Paromycin, Streptomycin, Tobramycin, Viomycin, Butirosin, Netilmicin oder Sisomycin ist.

4. Das Verfahren des Anspruchs 1, wobei der genannte Hilfstoff Natriumsalicylat oder Natriummethoxysalicylat ist.

**Revendications pour les Etats contractants: BE CH DE FR GB IT LI LU NL SE**

1. Une forme médicamenteuse d'administration orale comprenant une quantité thérapeutique efficace d'un antibiotique de la famille des macrolides ou des aminosides et un adjuvant de formule

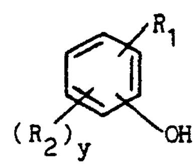

dans laquelle $R_1$ est $CO_2H$, $(CH_2)COOH$,

H
|
—C—$CO_2H$, $SO_3H$,
|
OH

ou un sel convenant en pharmacie correspondant, où $R_2$ est OH, H, un radical alcoxy inférieur ayant 1 à 10 atomes de carbone, un radical alkyle inférieur ayant 1 à 10 atomes de carbone, un radical halogèno ou un radical trigalogénoalkyle inférieur ayant 1 à 5 atomes de carbone, et où y est un entier de 1 ou 2.

2. La forme médicamenteuse de la revendication 1 dans laquelle ledit antibiotique de la famille des macrolides est l'érythromycine, l'oléandomycine, la spiramycine ou la kitasamycine.

3. La forme médicamenteuse de la revendication 1 dans laquelle l'antibiotique de la famille des aminosides est la gentamicine, la kanamycine, la néomycine, la paromycine, la streptomycine, la tobramycine, la viomycine, la butirosine, la nétilmicine ou la sisomycine.

4. La forme médicamenteuse de la revendication 1 dans laquelle ledit adjuvant est le salicylate de sodium ou le méthoxysalicylate de sodium.

**Revendications pour l'Etat contractant: AT**

1. Un procédé pour accroître la vitesse d'absorption dans la circulation sanguine d'un antibiotique de la famille des macrolides ou des aminosides administré par voie orale, ledit procédé comprenant les stades de préparation d'une forme médicamenteuse pouvant être absorbée par voie orale, ladite forme médicamenteuse comprenant une quantité thérapeutique efficace d'un antibiotique de la famille des macrolides ou des aminosides et un adjuvant de formule

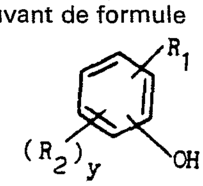

dans laquelle $R_1$ est $CO_2H$, $(CH_2)COOH$,

H
|
—C—$CO_2H$, $SO_3H$,
|
OH

ou un sel convenant en pharmacie correspondant,

où $R_2$ est OH, H, un radical alcoxy inférieur ayant 1 à 10 atomes de carbone, un radical alkyle inférieur ayant 1 à 10 atomes de carbone, un radical halogéno ou un radical trihalogénoalkyle inférieur ayant 2 à 5 atomes de carbone et où y est un entier de 1 ou 2, ledit adjuvant étant présent dans ladite forme médicamenteuse en une quantité suffisante pour être effiface pour accroître ladite vitesse d'absorption orale.

2. Le procédé de la revendication 1 dans lequel ledit antibiotique de la famille des macrolides est l'érythromycine, l'oléandomycine, la spiramycine ou la kitasamycine.

3. Le procédé de la revendication 1 dans lequel ledit antibiotique de la famille des aminosides est la gentamicine, la kanamycine, la néomycine, la paromycine, la streptomycine, la tobramycine, la viomycine, la butirosine, la nétilmicine ou la sisomycine.

4. Le procédé de la revendication 1 dans lequel ledit adjuvant est le salicylate de sodium ou le méthoxysalicylate de sodium.